# EUROPEAN PATENT SPECIFICATION

(11) **EP 1 437 158 B1**
(45) Date of publication and mention of the grant of the patent: **13.09.2006**
(21) Application number: 03078733.7
(22) Date of filing: 27.11.2003
(51) Int. Cl.: A61N 1/16, E04C 2/296

(54) **Mobile medical examination apparatus**
Mobile Vorrichtung zur medizinischen Untersuchung
Dispositif mobile destiné aux examens médicaux

(30) Priority: 10.01.2003 NL 1022355
(43) Date of publication of application: 14.07.2004
(73) Proprietor: F & J Holding B.V., 2718 RE Zoetermeer (NL)
(72) Inventor: Lamboo, Joost, 2731 BA Benthuizen (NL)
(74) Representative: Prins, Adrianus Willem

(56) References cited:
- DE-A- 3 628 494
- DE-A- 3 707 238
- FR-A- 1 318 217
- FR-A- 2 580 180
- US-A- 4 181 347

## Description

The invention relates to a mobile medical examination apparatus with an examination room for examination and/or treatment of patients by means of magnetic fields and/or particle radiation, comprising a number of walls, which are at least partly provided with shielding material which at least partly blocks the electromagnetic fields and/or particle radiation, wherein said walls comprise layered plates.

Such apparatuses are known from practice, see e.g. US-A-4 181 347, and usually comprise an examination apparatus built up on a mobile undercarriage. The undercarriage may, for instance, be a truck undercarriage, a trailer or a semitrailer. An advantage of a mobile apparatus is that it may, for instance, be used by a number of scattered hospitals together. For this purpose, the apparatus can alternately be arranged at one hospital or another. Further, such mobile apparatuses are suitable for population screening. Military applications are also possible.

Other known apparatuses have walls built up from multiplex plates, on which laths have been screwed in spaced relation on the inside. On the laths, shielding material has been fixed. For examination or treatment by X-radiation, a lead layer is needed to prevent the X-radiation from spreading to outside the examination room. For MRI examinations, for limiting the magnetic fields generated thereby, a shielding layer consisting of steel plate is usual. Further, usually, on the outside, a copper layer is used to prevent electromagnetic interfering radiation from outside.

"Electromagnetic radiation" is understood to mean both purely electronic or purely magnetic fields and electromagnetic fields, i.e. fields with an electric and a magnetic component.

The walls built up from multiplex, laths and shielding material in the manner described hereinabove are finally provided with a finishing layer on the inside and the outside.

A drawback of the known walls is that building them up is very labor-intensive and takes up a lot of time. Another drawback is that the known walls are relatively heavy and thick. Since, for normal road transport, maximum dimensions for the height, length and width of a vehicle apply, a great wall thickness is directly at the expense of the inner space available.

It is the object of the invention to obviate the drawbacks described and, in general, to provide a mobile medical examination apparatus which, while preserving the desired shielding effect, has walls which can be built up simply and rapidly and which are relatively thin and light. For this purpose, according to the invention, a mobile apparatus of the above-described type is characterized in that at least a number of walls comprise prefabricated panels, which have been built up by means of two spaced apart layered plates, which form an outer and an inner wall plate and which are interconnected via plastic sections, with the space between the two plates having been filled with plastic foam, and with at least one of the layered plates comprising at least one layer which at least partly blocks a magnetic field generated in the examination room or particle radiation generated in the examination room, and with, further, at least one of the layered plates comprising at least one layer which at least partly blocks electromagnetic fields prevailing outside the examination room.

In the following, the invention will be described in more detail with reference to the appended drawing of an exemplary embodiment, in which drawing:
Fig. 1 diagrammatically shows, in side elevational view, an example of a mobile medical apparatus in which the invention can be used;
Fig. 2 diagrammatically shows, in top plan view, the apparatus of Fig. 1; and
Fig. 3 diagrammatically shows, in cross section, an example of a wall construction for an apparatus according to the invention.

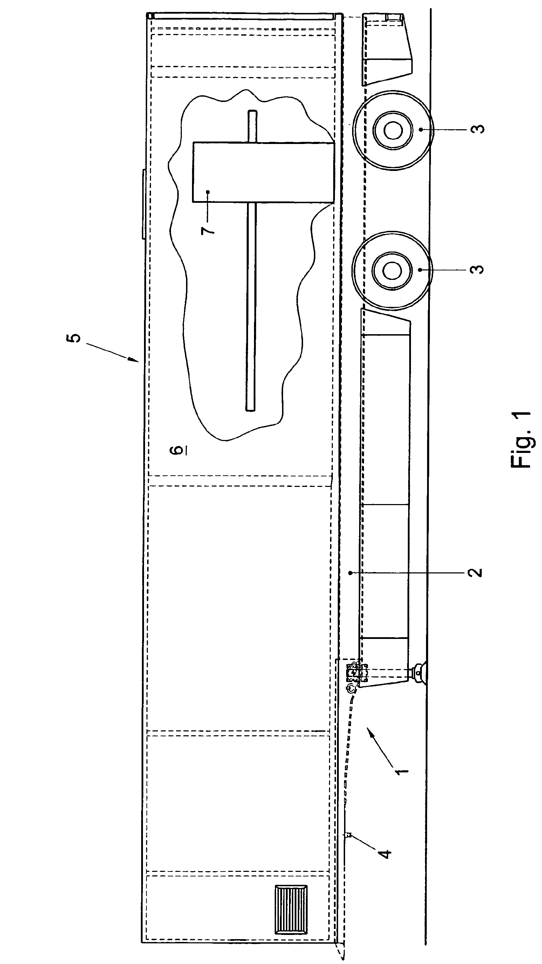

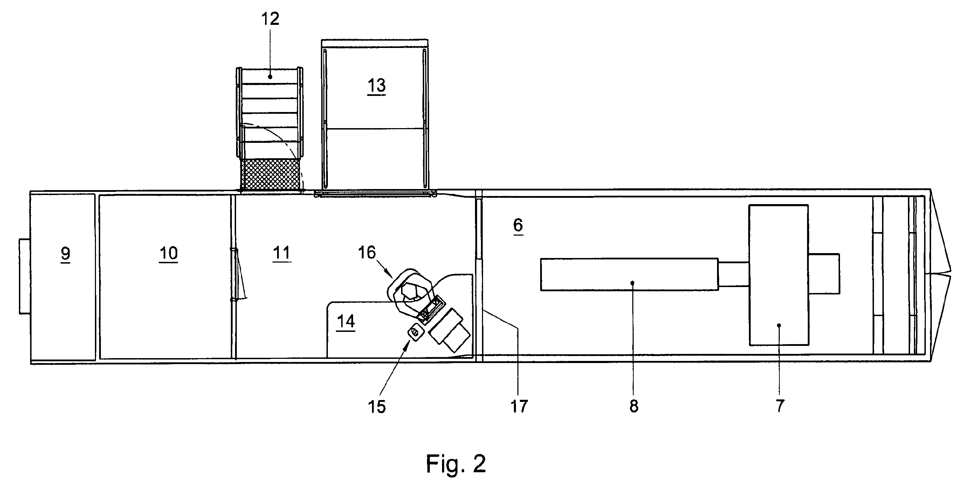

Fig. 1 and Fig. 2 diagrammatically show, in side elevational view and top plan view, an example of a mobile medical apparatus in which the invention can be used. The apparatus shown comprises a mobile undercarriage 1, which, in this example, has the form of a semitrailer. The semi-trailer has a chassis 2 with wheels 3 and a coupling pin 4 for cooperation with a fifth wheel or the like of a truck. Instead of a semitrailer, also, for instance, a truck bed or a pole trailer may be used. If desired, an undercarriage suitable for another type of transport, for instance a vessel undercarriage or the like, may also be used.

On the undercarriage, the actual medical apparatus 5 is provided. This comprises a number of rooms, including, in this example, an examination room 6, in which a medical device 7, such as for instance an X-radiation device or an MRI device, with a patient table 8 is provided. In the example shown, also, a technical room 9 and a dressing room 10 are provided, as well as a reception and control room 11, separated from the examination room by a partition wall 17, with access stairs 12 and a disabled elevator 13. In this example, in the reception and control room, further, a worktable 14 with a control station 15 for medical staff 16 is present.

The walls, including the ceiling and the floor, of at least the examination room should be at least partly provided with shielding material which at least partly blocks or absorbs radiation generated by the medical device 7. In the case of an MRI device, steel plates are used for this purpose, while, in the case of X-radiation equipment, lead is usually used. Further, to shield external electromagnetic fields and the like, often, copper foil is incorporated in the walls.

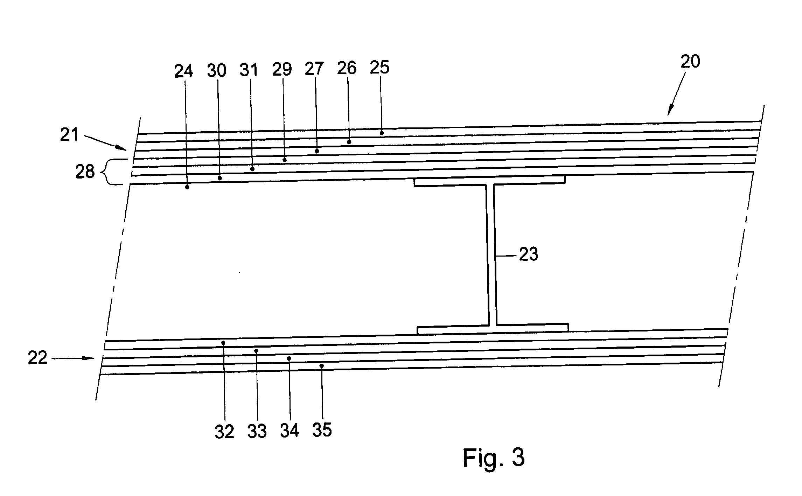

Fig. 3 diagrammatically shows, in cross section, an example of a wall construction 20 according to the invention. The wall construction shown is suitable for both functioning as radiation shield and functioning as construction element and comprises two spaced apart layered plates 21 and 22. The plates 21 and 22 are connected to each other via plastic I-sections 23, one of which is shown in Fig. 3. Further, the space between the layered plates and the 1-sections is filled with plastic foam 24, for instance hard foam attached to the plates. In practice, in this manner, wall panels can be manufactured having a length of the order of, for instance, 13.5 m and a height of 2.60 m.

It is noted that, in the drawing, the layers of plates 21 and 22 are all drawn equally thick. In reality, the layers have different thicknesses. The total wall thickness, i.e. the thickness of plates 21 and 22 as well as foam layer 24, may, in practice, be of the order of 11 to 14 cm, which is thin compared to the conventional constructions.

In the example shown, the outer plate 21 comprises the following layers from the outside to the inside. The outer layer 25 is a finishing layer, which may be, for instance, a polyester layer. The polyester layer is provided on a support layer 26 of plate material, which may, for instance, be multiplex. Then, an insulating layer 27 of insulating foam is provided. Next follows a composite magnetic shielding layer 28 built up from two layers 29,30 of magnetic shielding material which are provided on both sides of a non-compressible layer of non-magnetic plate material 31. The plate material may again be multiplex and the magnetic shielding material may be steel.

The various layers are glued and pressed on one another. The same holds true for the layers of the inner plate 22 to be described below. In the example shown, from the outside to the inside, the inner plate 22 comprises a layer of plate material 32, for instance multiplex. Like layer 30 of the outer plate, this layer is connected with I-sections 23 and with foam layer 24. On the side of layer 32 remote from foam layer 24, there is a layer of shielding material 33. This layer forms a shield against RF radiation coming from the outside. In this example, layer 33 is covered by a plastic layer 34, for instance a polyester layer, which may, in turn, be provided with a finishing layer 35.

The layer shielding against radio frequency (RF) fields may comprise metal gauze or aluminum foil. Preferably, however, copper foil is used, which is soldered on all sides, resulting in an RF radiation-proof cage. A layer of copper foil may be built up from a number of sheets of copper foil of a smaller size than a wall panel which are soldered together along the circumferential edges. The RF-shielding layer is also present in the partition wall 17 between the reception and control room 11 and the examination room 6. In order not to electrically interrupt the shield at the location of an opening in the wall, for instance, a window of glass provided with metal gauze is used. The metal gauze is, in turn, connected to the rest of the shielding layer. In the same manner, in air passages, so-called RF grids are placed. A door or hatch or the like may be provided with contacts on all sides, for instance so-called finger contacts, which, in closed condition of the door or the hatch or the like, form an electric connection with the rest of the RF-shielding layer.

With respect to the magnetic shielding layer, it is noted that it is required in the outer walls and the floor of the examination room to comply with relevant government regulations. The top wall does not have to be provided with a magnetic shielding layer. The distance between the MRI device 7 and the partition wall 17 is usually so great that, in the partition wall, no magnetic shielding layer is necessary either.

As described in the foregoing, in the example shown, the magnetic shielding layer is a composite layer with two layers of steel plate separated by a layer of non-magnetic material, such as for instance multiplex. It is also possible to use a single layer of steel plate. It has been found that, in that case, for a same shielding effect, the thickness of the single steel plate needs to be greater than the total thickness of the steel plates of the composite layer. Of course, if desired, a composite layer may be used which comprises more than two layers of steel plate separated by intermediate layers. The intermediate layers may again comprise a suitable non-compressible material, such as for instance multiplex. It is also conceivable that at least some of the steel shielding plates used are separated from one another by a glue and/or lacquer coating or a plastic foil or the like.

The layers providing magnetic shielding may each be built up from a number of plates of a smaller size connected to one another. Further, the layers providing magnetic shielding are preferably included in the outer plate 21 to prevent these layers from being pulled loose by the strong magnetic field of an MRI device.

In the case that the medical equipment comprises a device generating a particle radiation, such as for instance an X-radiation device, instead of one or more steel shielding layers, a shield built up from lead plates is needed. The lead plates are then preferably also provided on both sides of a suitable support material, such as for instance multiplex, resulting in a composite layer similar to the composite layer 28 of Fig. 3. Also in this case, variants with a single lead layer or, conversely, with a larger number of lead layers are possible.

It is noted that after the following, various modifications are obvious to a person skilled in the art. For instance, the layer providing shielding of external fields, such a radio transmitters, relay transmitters for mobile telephone systems, etc., may also be built up from multiple layers, optionally separated by intermediate layers. Further, if desired, it is possible to include the shielding layer for external fields in the outer wall plate. Such a construction can well be combined with, for instance, a lead layer in the inner wall plate.

It is further possible to use wall plates according to the invention in mobile apparatuses which are not or not permanently placed on an undercarriage.

In the wall plate filled with foam and the outer wall plate, tubes for electric wiring or medical gases and the like may advantageously be provided.

These and similar modifications are understood to be within the scope of the invention as determined by the claims.

## Claims

1. A mobile medical apparatus (5) with an examination room (6) for examination and/or treatment of patients by means of magnetic fields and/or particle radiation, comprising a number of walls (20), which are at least partly provided with shielding material which at least partly blocks electromagnetic fields and/or particle radiation, said walls comprising layered plates, **characterized in that** at least a number of walls comprise prefabricated panels, which have been built up by means of two spaced apart layered plates (21,22), which form an outer and an inner wall plate and which are interconnected via plastic sections (23), wherein the space between the two plates has been filled with plastic foam (24), and wherein at least one of the layered plates comprises at least one layer (28) which at least partly blocks a magnetic field generated in the examination room or particle radiation generated in the examination room, and wherein, further, at least one of the layered plates comprises at least one layer which at least partly blocks electromagnetic fields prevailing outside the examination room.

2. A mobile medical apparatus according to claim 1, **characterized in that** the at least one layer which at least partly blocks a magnetic field comprises a number of sublayers.

3. A mobile medical apparatus according to claim 2, **characterized in that** the sublayers comprise at least two layers of steel plate provided on both sides of a support layer of substantially non-compressible material.

4. A mobile medical apparatus according to claim 3, **characterized in that** the support layer comprises a multiplex plate.

5. A mobile medical apparatus according to claim 4, **characterized in that** the layer which blocks electromagnetic fields prevailing outside the examination room is a copper layer.

6. A mobile medical apparatus according to claim 5, **characterized in that** the copper layer is built up from sheets of copper foil soldered together.

7. A mobile medical apparatus according to any one of the preceding claims, **characterized in that** the at least one layer which at least partly blocks particle radiation comprises at least one lead layer.

8. A mobile medical apparatus according to claim 7, **characterized in that** the at least one layer which at least partly blocks particle radiation comprises at least two layers of lead provided on both sides of a support layer of substantially non-compressible material.

9. A mobile medical apparatus according to any one of the preceding claims, **characterized in that** the outer plate comprises a layer of insulating foam.

10. A mobile medical apparatus according to any one of the preceding claims, **characterized in that** the inner plate comprises a layer of polyester.

11. A mobile medical apparatus according to any one of the preceding claims, **characterized in that** the at least one layer which at least partly blocks magnetic fields and/or particle radiation generated in the examination room is located in the outer wall plate.

12. A mobile medical apparatus according to any one of the preceding claims, **characterized in that** the at least one layer which at least partly blocks electromagnetic fields prevailing outside the examination room is located in the inner wall plate.

13. A mobile medical apparatus according to any one of the preceding claims, **characterized in that** the foam in the space between the two wall plates is hard foam.

14. A mobile medical apparatus according to any one of the preceding claims, **characterized in that**, from the outside to the inside, the outer wall plate comprises the following successive layers: a polyester layer, a multiplex layer, a layer of insulating foam and a composite magnetic shielding layer.

15. A mobile medical apparatus according to any one of the preceding claims, **characterized in that**, from the outside to the inside, the inner wall plate comprises the following successive layers: a multiplex layer, a shielding layer for external electromagnetic fields, a polyester layer and an interior finishing layer.

16. A mobile medical apparatus according to any one of the preceding claims, **characterized in that** the layers of the inner and outer wall plates, respectively, are fixed on one another by means of glue and a pressing operation.

17. A prefabricated wall panel for use in a mobile medical apparatus according to any one of the preceding claims.

## Patentansprüche

1. Mobile medizinische Vorrichtung (5) mit einem Untersuchungsraum (6) zur Untersuchung und/oder Behandlung von Patienten mit Hilfe von Magnetfeldern und/oder Teilchenstrahlung, umfassend eine Anzahl von Wänden (20), die mindestens teilweise mit einem Abschirmungsmaterial versehen sind, welches mindestens teilweise elektromagnetische Felder und/oder Teilchenstrahlung blockiert, wobei die Wände geschichtete Platten umfassen, **dadurch gekennzeichnet, dass** mindestens eine Anzahl von Wänden vorgefertigte Paneele umfasst, die mit Hilfe von zwei beabstandeten geschichteten Platten (21, 22) aufgebaut wurden, die eine äußere und eine innere Wandplatte bilden und die über Kunststoffabschnitte (23) miteinander verbunden sind, wobei der Raum zwischen den beiden Platten mit Kunststoffschaum (24) gefüllt wurde, und wobei mindestens eine der geschichteten Platten mindestens eine Schicht (28) umfasst, die mindestens teilweise ein im Untersuchungsraum erzeugtes Magnetfeld oder im Untersuchungsraum erzeugte Teilchenstrahlung blockiert, und wobei des Weiteren mindestens eine der geschichteten Platten mindestens eine Schicht umfasst, die mindestens teilweise außerhalb des Untersuchungsraums herrschende elektromagnetische Felder blockiert.

2. Mobile medizinische Vorrichtung nach Anspruch 1, **dadurch gekennzeichnet, dass** die mindestens eine Schicht, die mindestens teilweise ein Magnetfeld blockiert, eine Anzahl von Unterschichten umfasst.

3. Mobile medizinische Vorrichtung nach Anspruch 2, **dadurch gekennzeichnet, dass** die Unterschichten mindestens zwei Schichten aus Stahlplatten umfassen, die an beiden Seiten einer tragenden Schicht aus im Wesentlichen nicht komprimierbarem Material vorgesehen sind.

4. Mobile medizinische Vorrichtung nach Anspruch 3, **dadurch gekennzeichnet, dass** die tragende Schicht eine Multiplex-Platte umfasst.

5. Mobile medizinische Vorrichtung nach Anspruch 4, **dadurch gekennzeichnet, dass** die Schicht, die außerhalb des Untersuchungsraums herrschende elektromagnetische Felder blockiert, eine Kupferschicht ist.

6. Mobile medizinische Vorrichtung nach Anspruch 5, **dadurch gekennzeichnet, dass** die Kupferschicht aus Lagen von zusammengelöteter Kupferfolie aufgebaut ist.

7. Mobile medizinische Vorrichtung nach einem der vorangehenden Ansprüche, **dadurch gekennzeichnet, dass** die mindestens eine Schicht, die mindestens teilweise Teilchenstrahlung blockiert, mindestens eine Bleischicht umfasst.

8. Mobile medizinische Vorrichtung nach Anspruch 7, **dadurch gekennzeichnet, dass** die mindestens eine Schicht, die mindestens teilweise Teilchenstrahlung blockiert, mindestens zwei Bleischichten umfasst, die an beiden Seiten einer tragenden Schicht aus im Wesentlichen nicht komprimierbarem Material vorgesehen ist.

9. Mobile medizinische Vorrichtung nach einem der vorangehenden Ansprüche, **dadurch gekennzeichnet, dass** die äußere Platte eine Schicht aus Isolierschaum umfasst.

10. Mobile medizinische Vorrichtung nach einem der vorangehenden Ansprüche, **dadurch gekennzeichnet, dass** die innere Platte eine Schicht aus Polyester umfasst.

11. Mobile medizinische Vorrichtung nach einem der vorangehenden Ansprüche, **dadurch gekennzeichnet, dass** die mindestens eine Schicht, die mindestens teilweise im Untersuchungsraum erzeugte Magnetfelder und/oder Teilchenstrahlung blockiert, in der äußeren Wandplatte liegt.

12. Mobile medizinische Vorrichtung nach einem der vorangehenden Ansprüche, **dadurch gekennzeichnet, dass** die mindestens eine Schicht, die mindestens teilweise außerhalb des Untersuchungsraums vorherrschende Magnetfelder blockiert, in der inneren Wandplatte liegt.

13. Mobile medizinische Vorrichtung nach einem der vorangehenden Ansprüche, **dadurch gekennzeichnet, dass** der Schaumstoff in dem Raum zwischen den beiden Wandplatten ein harter Schaumstoff ist.

14. Mobile medizinische Vorrichtung nach einem der vorangehenden Ansprüche, **dadurch gekennzeichnet, dass** die äußere Wand von außen nach innen die folgenden aufeinanderfolgenden Schichten umfasst: eine Polyesterschicht, eine Multiplexschicht, eine Schicht aus Isolierschaum und eine magnetisch abschirmende Verbundschicht.

15. Mobile medizinische Vorrichtung nach einem der vorangehenden Ansprüche, **dadurch gekennzeichnet, dass** die Innenwand von außen nach innen die folgenden aufeinanderfolgenden Schichten umfasst: eine Multiplexschicht, eine Abschirmungsschicht für externe elektromagnetische Felder, eine Polyesterschicht und eine innere Deckschicht.

16. Mobile medizinische Vorrichtung nach einem der vorangehenden Ansprüche, **dadurch gekennzeichnet, dass** die Schichten der inneren und äußeren Wandplatten jeweils mit Hilfe von Klebstoff und einem Pressvorgang aneinander befestigt sind.

17. Vorgefertigtes Wandpaneel zur Verwendung in einer mobilen medizinischen Vorrichtung nach einem der vorangehenden Ansprüche.

## Revendications

1. Dispositif médical mobile (5) avec une chambre d'examen (6) pour l'examen et/ou le traitement de patients au moyen de champs magnétiques et/ou de rayonnement particulaire, comprenant un nombre de parois (20), qui sont au moins en partie munies de matériau de blindage qui bloque au moins en partie les champs électromagnétiques et/ou le rayonnement particulaire, lesdites parois comprenant des plaques en couches, **caractérisé en ce qu'**au moins un nombre de parois comprennent des panneaux préfabriqués, qui ont été construits au moyen de deux plaques en couches espacées (21, 22), qui forment une plaque de paroi externe et interne et qui sont interconnectées via des sections en plastique (23), dans lequel l'espace entre les deux plaques a été rempli de mousse en plastique (24), et dans lequel au moins une des plaques en couches comprend au moins une couche (28) qui bloque au moins en partie un champ magnétique généré dans la chambre d'examen ou le rayonnement particulaire généré dans la chambre d'examen, et dans lequel, en outre, au moins une des plaques en couches comprend au moins une couche qui bloque au moins en partie les champs électromagnétiques prédominant à l'extérieur de la chambre d'examen.

2. Dispositif médical mobile selon la revendication 1, **caractérisé en ce qu'**au moins une couche qui bloque au moins en partie un champ magnétique comprend un nombre de sous-couches.

3. Dispositif médical mobile selon la revendication 2, **caractérisé en ce que** les sous-couches comprennent au moins deux couches de plaque d'acier fournies sur les deux côtés d'une couche de support de matériau essentiellement non compressible.

4. Dispositif médical mobile selon la revendication 3, **caractérisé en ce que** la couche de support comprend une plaque multiplex.

5. Dispositif médical mobile selon la revendication 4, **caractérisé en ce que** la couche qui bloque les champs électromagnétiques prédominant à l'extérieur de la chambre d'examen est une couche de cuivre.

6. Dispositif médical mobile selon la revendication 5, **caractérisé en ce que** la couche de cuivre est fabriquée à partir de feuillets de feuilles de cuivre soudées ensemble.

7. Dispositif médical mobile selon l'une quelconque des revendications précédentes, **caractérisé en ce que** l'au moins une couche qui bloque au moins en partie le rayonnement particulaire comprend au moins une couche de plomb.

8. Dispositif médical mobile selon la revendication 7, **caractérisé en ce qu'**au moins une couche qui bloque au moins en partie le rayonnement particulaire comprend au moins deux couches de plomb fournies sur les deux côtés d'une couche de support d'un matériau essentiellement non compressible.

9. Dispositif médical mobile selon l'une quelconque des revendications précédentes, **caractérisé en ce que** la plaque externe comprend une couche de mousse isolante.

10. Dispositif médical mobile selon l'une quelconque des revendications précédentes, **caractérisé en ce que** la plaque interne comprend une couche de polyester.

11. Dispositif médical mobile selon l'une quelconque des revendications précédentes, **caractérisé en ce que** l'au moins une couche qui bloque au moins en partie les champs magnétiques et/ou le rayonnement particulaire généré(s) dans la salle d'examen est située dans la plaque de paroi externe.

12. Dispositif médical mobile selon l'une quelconque des revendications précédentes, **caractérisé en ce que** l'au moins une couche qui bloque au moins en partie les champs électromagnétiques prédominant à l'extérieur de la chambre d'examen est située dans la plaque de paroi interne.

13. Dispositif médical mobile selon l'une quelconque des revendications précédentes, **caractérisé en ce que** la mousse dans l'espace entre les deux plaques de paroi est de la mousse dure.

14. Dispositif médical mobile selon l'une quelconque des revendications précédentes, **caractérisé en ce que**, de l'extérieur vers l'intérieur, la plaque de paroi externe comprend les couches successives suivantes : une couche de polyester, une couche multiplex, une couche de mousse isolante et une couche de blindage magnétique composite.

15. Dispositif médical mobile selon l'une quelconque des revendications précédentes, **caractérisé en ce que**, de l'extérieur vers l'intérieur, la plaque de paroi interne comprend les couches successives suivantes : une couche multiplex, une couche de blindage pour les champs magnétiques externes, une couche de polyester et une couche de finissage intérieur.

16. Dispositif médical mobile selon l'une quelconque des revendications précédentes, **caractérisé en ce que** les couches des plaques de paroi interne et externe, respectivement, sont fixées l'une sur l'autre au moyen de colle et d'une opération de pressage.

17. Panneau de paroi préfabriqué à utiliser dans un dispositif médical mobile selon l'une quelconque des revendications précédentes.
